# EUROPEAN PATENT APPLICATION

(11) **EP 4 011 452 A1**
(43) Date of publication of application: **15.06.2022**
(21) Application number: 20850715.2
(22) Date of filing: 07.08.2020
(51) Int. Cl.: A61P 35/00, A61P 35/02, A61K 31/443

(54) **THERAPEUTIC AGENT FOR CANCER HAVING RESISTANCE TO ANTI-CCR4 ANTIBODY**

(30) Priority: 08.08.2019 JP 2019146701
(71) Applicant: Daiichi Sankyo Company, Limited, Tokyo 103-8426 (JP)
(72) Inventor: FUJITANI Sumiaki, Tokyo 103-8426 (JP)
(74) Representative: Fairbairn, Angus Chisholm
(86) International application number: PCT/JP2020/030363
(87) International publication number: WO 2021/025148

(57) **Abstract**

An object of the present invention is to provide a therapeutic agent having an excellent effect on ATL patients with a history of treatment with an anti-CCR4 antibody, in other words, ATL patients who developed resistance to an anti-CCR4 antibody. An EZH1/2 dual inhibitor, a compound represented by Formula (I) or a pharmaceutically acceptable salt thereof was found to have an excellent effect on anti-CCR4 antibody-resistant cancer (particularly mogamulizumab-resistant cancer).

## Description

### Technical Field

The present invention relates to a therapeutic agent for anti-CCR4 antibody-resistant cancer, comprising a compound represented by Formula (I) or a pharmaceutically acceptable salt thereof as an active ingredient, particularly to a therapeutic agent for mogamulizumab-resistant cancer.

### Background Art

Adult T-cell leukemia/lymphoma (ATL) is cancer caused by a plurality of genetic abnormalities accumulated in peripheral blood T-cells infected with HTLV-1 during a latent period of as long as 50 to 60 years. Currently, it is estimated that 20 million people (carriers) are infected with human T-cell leukemia virus type I (HTLV-1) in the world. In Japan, it is estimated that at least 1.08 million people are infected with HTLV-1 and conceivably about 1200 people per year develop ATL (Non-patent Literature 1). There are many unclear points regarding the molecular mechanisms underlying cellular immortalization and tumorigenesis with HTLV-1, and treatment resistance. Effective therapies for ATL and methods for selectively removing infected cells have not yet been found. Treatment outcomes have improved with the development of an anti-CCR4 antibody as a molecular target drug against ATL, but prognosis of ATL is still the poorest among the malignant lymphomas (Non-patent Literature 3). The treatment outcome of chemotherapy for acute ATL, i.e., median overall survival time, is 12.7 months (Non-patent Literature 4), whereas the treatment outcome by the anti-CCR4 antibody against recurrence of ATL, i.e., median overall survival time, is 13.7 months (Non-patent Literature 5). Therefore, the prevention of viral infection and onset of leukemia, and the development of new therapies based on elucidation of the pathophysiology at the molecular level are urgent.

It has been revealed, as a result of large scale analysis of gene expression using a large number of ATL clinical samples, that ATL cells are a population having an extremely uniform and abnormal gene expression pattern (Non-patent Literature 6). Moreover, ATL cells have a characteristic signal transduction system abnormality, which corresponds to a key of survival and growth of tumor cells, and it has been revealed that the abnormality is derived from accumulation of epigenetic abnormalities (Non-patent Literature 7).

The polycomb family down-regulates gene expression by modulating chromatin via histone modification. Enhancer of Zeste Homologue 1/2 (EZH1/2) is an active center of Polycomb Repressive Complex 2 (PRC2) that catalyzes trimethylation of histone H3K27. EZH1 and EZH2 compensate each other for their functions to maintain an epigenome within a cell. Inhibition of EZH2 leads to a reduction in methylation level of H3K27 throughout the cell but this inhibitory effect is limited by the compensating effect of EZH1. When EZH1 and EZH2 are simultaneously inhibited, methylation can be more effectively inhibited (Non-patent Literature 8). It has been found that abnormality in a PRC2 component leads to cancer or abnormality in stem cell function, and in particular, accumulation of methylated H3K27me3 induced by gene abnormality or increased expression of EZH2 is identified in a large number of cancers, and earnest studies are being conducted mainly for EZH2 as a novel cancer molecular target drug (Non-patent Literatures 9, 10) .

The abnormality of polycomb family occurring in ATL was cleared by exhaustive gene expression analysis (Non-patent Literatures 6, 11). Of the abnormalities, overexpression of EZH2 is conspicuous, and increase of the methylation level of H3K27 in the entire cells is also detected. Besides, it has been found that EZH2-dependent inhibition of miR-31 results in expression of NF-**κ**B Inducing Kinase (NIK) so as to constitutively activate the NF-**κ**B pathway. Thus, EZH2 is considered effective as a molecular target for ATL. As an EZH1/2 dual inhibitor, (2R)-7-chloro-2-[trans-4-(dimethylamino)cyclohexyl]-N-[(4,6-dimethyl-2-oxo-1,2-dihydropyridin-3-yl)methyl]-2,4-dimethyl-1,3-benzodioxol-5-carboxamide and a pharmaceutically acceptable salt thereof, are known (Patent Literatures 1, 2).

At present, an effective therapy for ATL patients who developed resistance to an anti-CCR4 antibody has not yet been established. For example, it is reported that the response rate of ATL patients with a history of treatment with the anti-CCR4 antibody mogamulizumab, to the immunomodulator lenalidomide, which was approved in 2017 to be indicated for recurrent or refractory ATL, is about 18%, in phase II trials (Non-patent Literature 12).

### Citation List

### Patent Literatures

Patent Literature 1: WO2015141616
Patent Literature 2: WO2017018499

### Non-patent Literatures

Non-patent Literature 1: Yamaguchi K, Watanabe T., Int J Hematol 2002; 76 Suppl 2: 240.
Non-patent Literature 2: Iwanaga, M et al., Blood 2010; 116 (8): 1211-9.
Non-patent Literature 3: Vose, Armitage, J et al., J Clin Oncol 2008; 26 (25): 4124-30.
Non-patent Literature 4: Utsunomiya, A et. al., J Clin Oncol 2007; 25 (34): 5458-64.
Non-patent Literature 5: Ishida, Joh, et. al., J Clin Oncol 2012; 30 (8): 837-42.
Non-patent Literature 6: Yamagishi M et al., Cancer Cell 2012; 21: 121.
Non-patent Literature 7: Yamagishi M, Watanabe T., Front Microbiol 2012; 3: 334.
Non-patent Literature 8: Shen, X et al., Mol Cell 2008; 32 (4): 491-502.
Non-patent Literature 9: Sparmann A, van Lohuizen M., Nat Rev Cancer 2006; 6: 846.
Non-patent Literature 10: Lund, Adams, Copland., Leukemia 2014; 28 (1): 44-9.
Non-patent Literature 11: Sasaki D, et al., Haematologica 2011; 96: 712.
Non-patent Literature 12: Ishida T, et al., J Clin Oncol 2016; 34 (34): 4086-4093.

### Summary of Invention

### Technical Problem

The present invention was achieved in consideration of the aforementioned circumstances. An object of the invention is to provide a therapeutic agent having an excellent effect on ATL patients with a history of treatment with an anti-CCR4 antibody, in other words, ATL patients who developed resistance to an anti-CCR4 antibody.

### Solution to Problem

The present inventors conducted intensive studies with a view to achieving the object. As a result, they have found that an EZH1/2 dual inhibitor, a compound represented by Formula (I) or a pharmaceutically acceptable salt thereof, has an excellent effect on anti-CCR4 antibody-resistant cancer (particularly mogamulizumab-resistant cancer). Based on this finding, they have accomplished the present invention. The present invention relates to the following (1) to (12).

(1) A therapeutic agent for anti-CCR4 antibody-resistant cancer, comprising a compound represented by the following Formula (I): 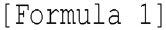 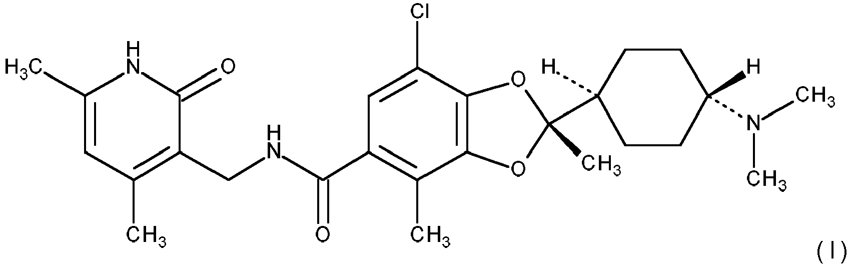 or a pharmaceutically acceptable salt thereof as an active ingredient.
(2) The therapeutic agent according to (1), wherein the anti-CCR4 antibody is mogamulizumab.
(3) The therapeutic agent according to (1) or (2), wherein the cancer is adult T cell leukemia/lymphoma.
(4) A method for treating anti-CCR4 antibody-resistant cancer in patients with anti-CCR4 antibody-resistant cancer, comprising administering a compound represented by Formula (I) or a pharmaceutically acceptable salt thereof.
(5) The method according to (4), wherein the anti-CCR4 antibody is mogamulizumab.
(6) The method according to (4) or (5), wherein the cancer is adult T cell leukemia/lymphoma.
(7) A compound represented by Formula (I) or a pharmaceutically acceptable salt thereof for treating anti-CCR4 antibody-resistant cancer.
(8) The compound or a pharmaceutically acceptable salt thereof according to (7), wherein the anti-CCR4 antibody is mogamulizumab.
(9) The compound or a pharmaceutically acceptable salt thereof according to (7) or (8), wherein the cancer is adult T cell leukemia/lymphoma.
(10) Use of a compound represented by Formula (I) or a pharmaceutically acceptable salt thereof in producing a pharmaceutical composition for treating anti-CCR4 antibody-resistant cancer.
(11) The use according to (10), wherein the anti-CCR4 antibody is mogamulizumab.
(12) The use according to (10) or (11), wherein the cancer is adult T cell leukemia/lymphoma.

Another aspect of the present invention relates to the following (1) to (9).

(1) A therapeutic agent for anti-CCR4 antibody-resistant cancer, comprising (2R)-7-chloro-2-[trans-4-(dimethylamino)cyclohexyl]-N-[(4,6-dimethyl-2-oxo-1,2-dihydropyridin-3-yl)methyl]-2,4-dimethyl-1,3-benzodioxol-5-carboxamide or a pharmaceutically acceptable salt thereof as an active ingredient.
(2) A therapeutic agent for anti-CCR4 antibody-resistant cancer, comprising (2R)-7-chloro-2-[trans-4-(dimethylamino)cyclohexyl]-N-[(4,6-dimethyl-2-oxo-1,2-dihydropyridin-3-yl)methyl]-2,4-dimethyl-1,3-benzodioxol-5-carboxamide p-toluenesulfonate as an active ingredient.
(3) The therapeutic agent according to (1) or (2), wherein the anti-CCR4 antibody is mogamulizumab.
(4) The therapeutic agent according to any one of (1) to (3), wherein the cancer is adult T cell leukemia/lymphoma.
(5) A method for treating anti-CCR4 antibody-resistant cancer, comprising administering (2R)-7-chloro-2-[trans-4-(dimethylamino)cyclohexyl]-N-[(4,6-dimethyl-2-oxo-1,2-dihydropyridin-3-yl)methyl]-2,4-dimethyl-1,3-benzodioxol-5-carboxamide or a pharmaceutically acceptable salt thereof.
(6) A method for treating anti-CCR4 antibody-resistant cancer, comprising administering (2R)-7-chloro-2-[trans-4-(dimethylamino)cyclohexyl]-N-[(4,6-dimethyl-2-oxo-1,2-dihydropyridin-3-yl)methyl]-2,4-dimethyl-1,3-benzodioxol-5-carboxamide p-toluenesulfonate.
(7) The method according to (5) or (6), wherein the anti-CCR4 antibody is mogamulizumab.
(8) The method according to any one of (5) to (7), wherein the cancer is adult T cell leukemia/lymphoma.
(9) (2R)-7-chloro-2-[trans-4-(dimethylamino)cyclohexyl]-N-[(4,6-dimethyl-2-oxo-1,2-dihydropyridin-3-yl)methyl]-2,4-dimethyl-1,3-benzodioxol-5-carboxamide or a pharmaceutically acceptable salt thereof for treating anti-CCR4 antibody-resistant cancer.
(10) (2R)-7-chloro-2-[trans-4-(dimethylamino)cyclohexyl]-N-[(4,6-dimethyl-2-oxo-1,2-dihydropyridin-3-yl)methyl]-2,4-dimethyl-1,3-benzodioxol-5-carboxamide p-toluenesulfonate for treating anti-CCR4 antibody-resistant cancer.
(11) The compound or a pharmaceutically acceptable salt thereof according to (9) or (10), wherein the anti-CCR4 antibody is mogamulizumab.
(12) The compound or a pharmaceutically acceptable salt thereof according to any one of (9) to (11), wherein the cancer is adult T cell leukemia/lymphoma.
(13) Use of (2R)-7-chloro-2-[trans-4-(dimethylamino)cyclohexyl]-N-[(4,6-dimethyl-2-oxo-1,2-dihydropyridin-3-yl)methyl]-2,4-dimethyl-1,3-benzodioxol-5-carboxamide or a pharmaceutically acceptable salt thereof in producing a pharmaceutical composition for treating anti-CCR4 antibody-resistant cancer.
(14) Use of (2R)-7-chloro-2-[trans-4-(dimethylamino)cyclohexyl]-N-[(4,6-dimethyl-2-oxo-1,2-dihydropyridin-3-yl)methyl]-2,4-dimethyl-1,3-benzodioxol-5-carboxamide p-toluenesulfonate in producing a pharmaceutical composition for treating anti-CCR4 antibody-resistant cancer.
(15) The use according to (13) or (14), wherein the anti-CCR4 antibody is mogamulizumab.
(16) The use according to any one of (13) to (15), wherein the cancer is adult T cell leukemia/lymphoma.

### Advantageous Effects of Invention

The present invention enables the provision of a therapeutic agent having an excellent effect on ATL patients with a history of treatment with an anti-CCR4 antibody, in other words, ATL patients who developed resistance to anti-CCR4 antibody.

### Description of Embodiments

A compound represented by Formula (I) of the present invention has the following chemical structure and is referred to as (2R)-7-chloro-2-[trans-4-(dimethylamino)cyclohexyl]-N-[(4,6-dimethyl-2-oxo-1,2-dihydropyridin-3-yl)methyl]-2,4-dimethyl-1,3-benzodioxol-5-carboxamide. A compound represented by Formula (I) can be manufactured by a method described, for example, in WO2015141616.

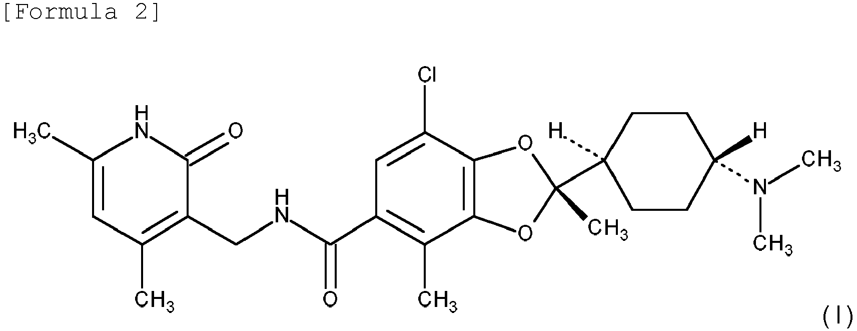

In the present invention, the term "pharmaceutically acceptable salt" refers to a salt that has no significant toxicity and can be used in a pharmaceutical composition. A compound represented by Formula (I) of the present invention can be reacted with an acid to form a salt. Examples of the salt include, but are not limited to, inorganic acid salts including a salt of a hydrohalic acid, such as a salt of hydrofluoric acid, a hydrochloride, a salt of hydrobromic acid and a salt of hydroiodic acid, a nitrate, a perchlorate, a sulfate and a phosphate; organic acid salts including a C₁-C₆ alkyl sulfonate, such as methanesulfonate, trifluoromethanesulfonate and ethane sulfonate, an aryl sulfonate such as benzene sulfonate and p-toluenesulfonate, an acetate, a malate, a fumarate, a succinate, a citrate, an ascorbate, a tartrate, an oxalate, an adipate; and amino acid salts such as a glycine salt, a lysine salt, an arginine salt, an ornithine salt, a glutamate acid and aspartate.

A pharmaceutically acceptable salt of a compound represented by Formula (I) of the present invention is most preferably (2R)-7-chloro-2-[trans-4-(dimethylamino)cyclohexyl]-N-[(4,6-dimethyl-2-oxo-1,2-dihydropyridin-3-yl)methyl]-2,4-dimethyl-1,3-benzodioxol-5-carboxamide p-toluenesulfonate (hereinafter referred to as the "clinical compound")

A compound represented by Formula (I) of the present invention or a pharmaceutically acceptable salt thereof sometimes takes up water molecules to form a hydrate when it is left in the air or recrystallized. Such a hydrate is encompassed in the present invention.

A compound represented by Formula (I) of the present invention or a pharmaceutically acceptable salt thereof sometimes absorbs a solvent to form a solvate when it is left in the solvent or recrystallized. Such a solvate is encompassed in the present invention.

In a compound represented by Formula (I) of the present invention or a pharmaceutically acceptable salt thereof, all isomers (e.g., a diastereomer, an enantiomer, a geometrical isomer, a rotamer) are included.

A compound represented by Formula (I) of the present invention, these isomers and mixtures of the isomers are all represented by a single formula. Thus, the present invention includes all of these isomers and mixtures of these isomers in any proportion.

In the specification, the term "cancer" refers to all malignant tumors.

Cancer can be classified into "solid cancer" and "blood cancer". Solid cancer can be classified into "epithelial cell cancer" and "non-epithelial cell cancer". Epithelial cell cancer refers to cancer developed from epithelial cells. Examples epithelial cell cancer include lung cancer, gastric cancer, liver cancer, kidney cancer, prostate cancer, pancreatic cancer, colorectal cancer, breast cancer and ovarian cancer. Non-epithelial cell cancer refers to cancer developed from non-epithelial cells such as bone and muscle. Examples of non-epithelial cell cancer include osteosarcoma, chondrosarcoma and rhabdomyosarcoma. Blood cancer refers to cancer developed from hematopoietic organs and is classified into, for example, malignant lymphoma, leukemia and multiple myeloma.

A target cancer to be treated in the present invention is preferably malignant lymphoma and leukemia, and further preferably, non-Hodgkin's lymphoma, and further more preferably, adult T cell leukemia/lymphoma.

In the present specification, the term "adult T cell leukemia/lymphoma" (hereinafter sometimes referred to simply as "ATL") refers to leukemia/lymphoma caused by infection with human T cell leukemia virus type 1 (HTLV-1). ATL is sometimes referred to as "adult T cell leukemia" or "adult T cell lymphoma".

ATL is diagnosed by a doctor based on clinical symptoms of a subject. ATL is a disease caused by the monoclonal proliferation of T cells in which HTLV-1 is integrated into the genome as a provirus. ATL is diagnosed by detecting monoclonal integration of a HTLV-1 provirus in the DNA of ATL cells in a sample taken from a subject, by, e.g., southern blotting.

In the present specification, the term "treating" and terms derived from this mean remission and alleviation of clinical symptoms of cancer and/or delay of exacerbation in a patient with cancer.

According to experimental examples described later, a compound represented by Formula (I) or a pharmaceutically acceptable salt thereof can reduce growth of anti-CCR4 antibody-resistant adult T cell leukemia/lymphoma; reduce the viability of anti-CCR4 antibody-resistant adult T cell leukemia/lymphoma; and/or kill anti-CCR4 antibody-resistant adult T cell leukemia/lymphoma. Thus, according to the present invention, provided is a composition or a pharmaceutical composition for use in reducing growth of anti-CCR4 antibody-resistant adult T cell leukemia/lymphoma; reducing the viability of anti-CCR4 antibody-resistant adult T cell leukemia/lymphoma; and/or killing anti-CCR4 antibody-resistant adult T cell leukemia/lymphoma, the composition comprising a compound represented by Formula (I) or a pharmaceutically acceptable salt thereof. According to the present invention, provided is use of a compound represented by Formula (I) or a pharmaceutically acceptable salt thereof in production of a composition or a pharmaceutical composition for use in reducing growth of anti-CCR4 antibody-resistant adult T cell leukemia/lymphoma; reducing the viability of anti-CCR4 antibody-resistant adult T cell leukemia/lymphoma; and/or killing anti-CCR4 antibody-resistant adult T cell leukemia/lymphoma. According to the present invention, provided is a method for reducing growth of anti-CCR4 antibody-resistant adult T cell leukemia/lymphoma; reducing the viability of anti-CCR4 antibody-resistant adult T cell leukemia/lymphoma; and/or killing anti-CCR4 antibody-resistant adult T cell leukemia/lymphoma in a subject in need thereof, the method comprising administering to the subject a compound represented by Formula (I) or a pharmaceutically acceptable salt thereof.

In the specification, the term "anti-CCR4 antibody" refers to an antibody to chemokine receptor type 4; for example, mogamulizumab is known.

In the specification, the term "anti-CCR4 antibody-resistant" refers to the state where a cancer (preferably adult T cell leukemia/lymphoma) no longer effectively responds to an anti-CCR4 antibody. The phrase "no longer effectively responds" refers to the state where cancer suppressed in growth by administration of an anti-CCR4 antibody initiates growth again despite of administration of the anti-CCR4 antibody. Whether cancer grows or not can be confirmed by a method ordinarily used in the technical field. Examples of the confirmation method, include a blood test or bone marrow examination for confirming an increase or not of abnormal T cells due to HTLV-1 virus infection in the blood or bone marrow, CT inspection for confirming swelling of lymph nodes, an endoscopic examination for confirming hepatosplenomegaly, and skin biopsy for confirming skin lesions.

In the specification, the phrase "with a history of treatment with an anti-CCR4 antibody" means that a treatment with an anti-CCR4 antibody was applied but terminated because the anti-CCR4 antibody was no longer effective. The phrase is synonymous with anti-CCR4 antibody-resistant.

In the specification, the term "mogamulizumab" is an anti-cancer agent classified as an anti-CCR4 antibody and used worldwide under the trade name "Poteligeo".

In the specification, the term "lenalidomide" is an anti-cancer agent classified as an immunomodulator and used worldwide under the trade name "Revlimid".

An aspect of the present invention relates to a therapeutic agent for lenalidomide-resistant cancer, comprising a compound represented by Formula (I) or a pharmaceutically acceptable salt thereof as an active ingredient, and preferably, a therapeutic agent for lenalidomide-resistant adult T cell leukemia/lymphoma, comprising a compound represented by Formula (I) or a pharmaceutically acceptable salt thereof as an active ingredient.

Another aspect of the present invention relates to a method for treating lenalidomide-resistant cancer, comprising administering a compound represented by Formula (I) or a pharmaceutically acceptable salt thereof; and preferably a method for treating lenalidomide-resistant adult T cell leukemia/lymphoma, comprising administering a compound represented by Formula (I) or a pharmaceutically acceptable salt thereof.

Another aspect of the present invention relates to a compound represented by Formula (I) or a pharmaceutically acceptable salt thereof for treating lenalidomide-resistant cancer; and preferably a compound represented by Formula (I) or a pharmaceutically acceptable salt thereof for treating lenalidomide-resistant adult T cell leukemia/lymphoma.

Another aspect of the present invention relates to use of a compound represented by Formula (I) or a pharmaceutically acceptable salt thereof in producing a pharmaceutical composition for treating lenalidomide-resistant cancer; and preferably use of a compound represented by Formula (I) or a pharmaceutically acceptable salt thereof in producing a pharmaceutical composition for treating lenalidomide-resistant adult T cell leukemia/lymphoma.

Another aspect of the present invention relates to a method for treating a recurrent or refractory adult T cell leukemia/lymphoma patient with a history of mogamulizumab use, or a recurrent or refractory adult T cell leukemia/lymphoma patient for whom mogamulizumab is not suitable and who has received one or more systemic chemotherapy regimens, by administering a compound represented by Formula (I) or a pharmaceutically acceptable salt thereof.

A compound represented by Formula (I) of the present invention or a pharmaceutically acceptable salt thereof may be used in combination with another anti-tumor agent and another therapy (for example, radiation therapy, immunotherapy).

When a compound represented by Formula (I) of the present invention or a pharmaceutically acceptable salt thereof is prepared as a pharmaceutical composition, a pharmaceutically acceptable carrier may be used. Examples of the carrier include, but are not limited to, sterilized water, saline, a vegetable oil, a solvent, a base, an emulsifier, a suspension, a surfactant, a stabilizer, a flavoring agent, an aromatic, an excipient, a vehicle, a preservative, a binder, a diluent, a tonicity agent, a soothing agent, a thickening agent, a disintegrant, a buffer, a coating agent, a lubricant, a colorant, a sweetener, a viscous agent, a corrigent and a dissolution aid. The compound of the present invention or a pharmaceutically acceptable salt thereof may take various dosage forms such as tablets, powders, granules, capsules and liquids, which are determined depending on the therapeutic purpose. Alternatively, a liposomal delivery system may be employed as a dosage form. To the liposomes, an adjuvant (for example, antibody and ligand) can be added for augmenting a therapeutically useful property.

An oral or parenteral administration to patients can be employed. Examples of the parenteral administration include, intravenous administration, arterial administration, intramuscular administration, intrathoracic administration, intraperitoneal administration, direct administration to a target site (for example, tumor).

The dosage amount is not limited as long as it is effective for treating the target disease and appropriately selected depending on the age, body weight, symptomatology, health and disease progression of the patient. The administration frequency is not particularly limited and appropriately selected depending on the purpose. For example, the daily dose may be administered once a day or divided into a plurality of portions and administered multiple times. When the medical agent of the present invention is administered to humans, the dosage amount of an active ingredient falls within the range of usually about 0.01 mg/kg body weight to about 500 mg/kg body weight, and preferably, about 0.1 mg/kg body weight to about 100 mg/kg body weight per day. When the medical agent of the present invention is administered to humans, the administration frequency is preferably, once a day, or the daily dose is divided into 2 to 4 portions and administration is preferably repeated at appropriate intervals.

Note that, when the compound of the present invention or a pharmaceutically acceptable salt thereof is prepared as a reagent, if necessary, other components acceptable as reagents, such as sterilized water, saline, a buffer and a preservative, may be included. If the reagents are administered to a subject (for example, cells, fraction thereof, tissue and experimental animals) in a dose according to the purpose, EZH1/2 can be inhibited to suppress tumor growth, for example.

### Examples

Now, the present invention will be more specifically described by way of Examples but the scope of the present invention is not limited by these Examples.

The abbreviation codes employed in the following Examples are defined as follows:
CR: Complete Remission
CRu: Uncertified Complete Remission
PR: Partial Remission
SD: Stable Disease
PD: Progressive Disease
PFS: Progression-Free Survival
NE: Not Evaluable

### Example 1 Administration of clinical compound to patients with non-Hodgkin's lymphoma

Clinical trials which administer the clinical compound to patients with non-Hodgkin's lymphoma were carried out. ATL patients of the non-Hodgkin's lymphoma patients receiving the clinical compound were analyzed.

### (Patients to be subjected to clinical trial)

Inclusion criteria:
   1) Patients hematologically or pathologically diagnosed as non-Hodgkin's lymphoma;
   2) Patients for whom no standard treatment existed or patients who became refractory or intolerant to a standard treatment;
   3) Patients whose ECOG performance status is 0 or 1; and
   4) Patients of 18 years old or more (USA), 20 years old or more (Japan).
Exclusion criteria:
   1) Patients having any one of the following previous diseases (within 6 months before registration) or complications;
   2) Diabetes, chronic congestive heart failure (NYHA Functional Classification, class III or more), unstable angina, angioplasty, stenting, or history of myocardial infarction, symptomatic or arrhythmia requiring treatment or asymptomatic persistent ventricular tachycardia (except asymptomatic controllable atrial fibrillation) and other diseases, that were poorly controlled as diagnosed by investigators or clinical research coordinators; and
   3) Patients having active tuberculosis diseases, herpes simplex, systemic mycosis and active infectious diseases requiring administration of an antibacterial agent/antiviral agent/antifungal agent/steroid.

### (Design for clinical trial)

The clinical trial is a multicenter open-label phase I trial for the purpose of studying, e.g., safety, tolerability, pharmacokinetics and anti-tumor effect, by administering the clinical compound to patients with recurrent or refractory non-Hodgkin's lymphoma (no standard treatment is established or refractory or intolerant to a standard treatment) including ATL patients. When the clinical compound is repeatedly administered, starting from a low dose, to test subjects who satisfy the inclusion criteria and do not violate the exclusion criteria, if tolerance is confirmed, the dosage of a next cohort increases in a stepwise way. In this manner, dose-limiting toxicity and recommended dose for the next phase are determined in the clinical trial.

The clinical compound was orally administered to individual test subjects once a day for consecutive days until unacceptable toxicity or disease progression was confirmed. Individual test subjects were observed for evaluation of safety until 30 days after the final administration day of the clinical compound or the day at which another anti-cancer drug treatment was started, whichever comes first. The anti-tumor effect was observed principally, at intervals of 8 weeks, and evaluated based on "Definition, prognostic factors, treatment, and response criteria of adult T-cell leukemia-lymphoma: a proposal from an international consensus meeting" (J Clin Oncol. 2009; 27[3]: 453-9).

The dose of the first cohort was determined to be 150 mg/time/day based on the results of a nonclinical study. The dose of the next cohort was determined by comprehensively evaluating the dose recommended by the modified continual reassessment method (mCRM) based on information of the presence or absence of dose-limited toxicity and all safety data (including adverse event except dose limiting toxicity and abnormal laboratory test values), pharmacokinetics, biomarkers, and effectiveness data obtained up to the time point, and discussing with a sponsors, investigators and external experts.

### (Dosage form, dosage amount, administration route)

The dosage amount of the clinical compound was in advance determined per cohort. As the clinical compound, two types of capsules different in dose were provided in combination. As the administration method, oral administration once a day was carried out for consecutive days.

### (Confirmation of anti-tumor effect)

The therapeutic effect on ATL was determined based on "Definition, prognostic factors, treatment, and response criteria of adult T-cell leukemia-lymphoma: a proposal from an international consensus meeting" (J Clin Oncol. 2009; 27[3]: 453-9). Before initiation of the clinical trial, necessary examinations, which were selected from CT scan, bone marrow examination, endoscopic inspection and blood test in accordance with the disease state of the test subject, were carried out to determine a target lesion. A change in the lesion was assessed principally at intervals of 8 weeks.

### (Test results)

In the intermediate data of the clinical trial, the response rate of recurrent or refractory ATL patients on which effectiveness can be evaluated was 44.4% (4 out of 9 patients [CRu: 1 patient, PR: 3 patients]) (95%CI: 13.7 to 78.8) and the disease control rate thereof (percentage of patients exerting an effect of SD or more) was 77.8% (7 out of 9 patients). Particularly, of 6 patients with a history of treatment with mogamulizumab, 3 patients were responders (CRu: 1 patient, PR: 2 patients) and 2 patients were evaluated as SD. The median value of PFS of 9 recurrent or refractory ATL patients was 16.1 weeks. According to the epidemiological data obtained from almost the same types of patients as the subject patients, the median survival period is 8.3 to 10.6 months (Katsuya H, et al. Blood. 2015; 126 (24): 2570-7). From this, it was considered that the clinical compound of the invention will be an option for a new therapy. The results are shown in Table 1.

**[Table 1]**

| No. | Dose | Treatment history | | Determination of effect on lesion site | | | | Best overall effect | Treatment period (days) |
|---|---|---|---|---|---|---|---|---|---|
| | | Mogamulizumab | Lenalidomide | Peripheral blood image | Skin lesion | Nodular lesion | Extranodal lesion | | |
| 1 | 150 mg | Present | Present | - | NE | PD | PD | PD | 56 |
| 2 | 150 mg | Present | - | - | - | Cru | PR | PR | 112 |
| 3 | 200 mg | - | - | CR | - | CR | SD | SD | 227 (ongoing) |
| 4 | 200 mg | Present | - | - | - | PD | PD | PD | 15 |
| 5 | 200 mg | Present | - | - | PR | - | - | PR | 185 (ongoing) |
| 6 | 200 mg | Present | - | SD | - | - | - | SD | 148 (ongoing) |
| 7 | 200 mg | Present | - | - | - | - | CRu | CRu | 98 |
| 8 | 200 mg | - | - | - | CR | - | - | CR | 70 |
| 9 | 200 mg | - | Present | PR | - | - | - | PR | 86 (ongoing) |

Example 2 Administration of clinical compound to recurrent or refractory ATL patients with a history of mogamulizumab use, or recurrent or refractory ATL patients for whom mogamulizumab treatment is not suitable and who have received one or more regimens of a systemic chemotherapy

### (Patients to be subjected to clinical trial)

Recurrent or refractory ATL patients with a history of mogamulizumab use, or recurrent or refractory ATL patients for whom mogamulizumab treatment is not suitable and who have received one or more regimens of a systemic chemotherapy

### (Dosage form, dosage amount, administration route)

As the dosage amount of the clinical compound, the most appropriate dosage amount determined based on the data so far obtained is selected and clearly described in an experimental protocol or the like. As the clinical compound, two types of tablets different in dose are provided in combination. As the administration method, oral administration once a day is carried out for consecutive days.

### (Confirmation of anti-tumor effect)

The therapeutic effect on ATL is determined based on "Definition, prognostic factors, treatment, and response criteria of adult T-cell leukemia-lymphoma: a proposal from an international consensus meeting" (J Clin Oncol. 2009; 27[3]: 453-9). Before initiation of the clinical trial, necessary examinations, which are selected from CT scan, bone marrow examination, endoscopic inspection and blood test in accordance with the disease state of the test subject, are carried out to determine a target lesion. A change in the lesion was assessed for diagnosis. In addition to determination of a doctor in charge, determination is carried out independently and integrally by setting a data review committee outside the company.

For example, response rate (total of complete remission: [CR] rate, uncertified complete remission [CRu] rate, and partial remission [PR] rate), response duration, progression-free survival (PFS) and overall survival (OS) are set as secondary evaluation items. In this manner, exploratory evaluation of effectiveness is carried out.

## Claims

1. A therapeutic agent for anti-CCR4 antibody-resistant cancer, comprising a compound represented by the following Formula (I): 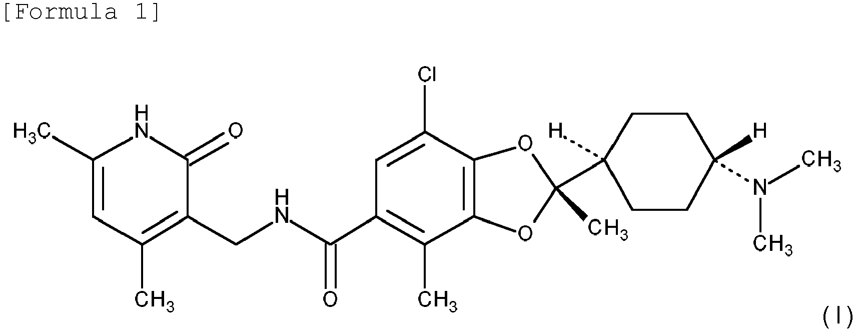 or a pharmaceutically acceptable salt thereof, as an active ingredient.

2. The therapeutic agent according to claim 1, wherein the anti-CCR4 antibody is mogamulizumab.

3. The therapeutic agent according to claim 1 or 2, wherein the cancer is adult T cell leukemia/lymphoma.

4. A method for treating anti-CCR4 antibody-resistant cancer in patients with anti-CCR4 antibody-resistant cancer, comprising administering a compound represented by Formula (I) or a pharmaceutically acceptable salt thereof.

5. The method according to claim 4, wherein the anti-CCR4 antibody is mogamulizumab.

6. The method according to claim 4 or 5, wherein the cancer is adult T cell leukemia/lymphoma.

7. A compound represented by Formula (I) or a pharmaceutically acceptable salt thereof, for treating anti-CCR4 antibody-resistant cancer.

8. The compound or a pharmaceutically acceptable salt thereof according to claim 7, wherein the anti-CCR4 antibody is mogamulizumab.

9. The compound or a pharmaceutically acceptable salt thereof according to claim 7 or 8, wherein the cancer is adult T cell leukemia/lymphoma.

10. Use of a compound represented by Formula (I) described above or a pharmaceutically acceptable salt thereof in producing a pharmaceutical composition for treating anti-CCR4 antibody-resistant cancer.

11. The use according to claim 10, wherein the anti-CCR4 antibody is mogamulizumab.

12. The use according to claim 10 or 11, wherein the cancer is adult T cell leukemia/lymphoma.
